Europäisches Patentamt

**(19)** European Patent Office

Office européen des brevets

**(11)** **EP 1 102 064 A1**

**(12)** ## DEMANDE DE BREVET EUROPEEN

**(43)** Date de publication:
**23.05.2001 Bulletin 2001/21**

**(51)** Int Cl.⁷: **G01N 33/18**

**(21)** Numéro de dépôt: **00402957.5**

**(22)** Date de dépôt: **25.10.2000**

**(84)** Etats contractants désignés:
**AT BE CH CY DE DK ES FI FR GB GR IE IT LI LU MC NL PT SE**
Etats d'extension désignés:
**AL LT LV MK RO SI**

**(30)** Priorité: **22.11.1999 FR 9914649**

**(71)** Demandeur: **L'air Liquide Société Anonyme pour l'étude et l'exploitation des procédés Georges Claude
75321 Paris Cédex 07 (FR)**

**(72)** Inventeurs:
• **Campo, Philippe
78180 Montigny le Bretonneux (FR)**

• **Grimberg, Aurélie
92210 Saint-Cloud (FR)**
• **Rabillier, Jean-Marc
78280 Guyancourt (FR)**
• **Rignon, Maurice
78280 Guyancourt (FR)**

**(74)** Mandataire: **Mellul-Bendelac, Sylvie Lisette et al L'Air Liquide,
Service Propriété Industrielle,
75, Quai d'Orsay
75321 Paris Cedex 07 (FR)**

**(54)** **Méthode de détermination d'un taux de traitement à l'ozone d'un liquide à traiter**

**(57)** L'invention concerne une méthode de détermination d'un taux de traitement à l'ozone d'un liquide à traiter (à partir de la détermination de la demande instantanée en ozone du liquide à traiter), selon laquelle on procède aux mesures suivantes :

a) on dispose d'une réserve dudit liquide à traiter, dont la teneur initiale en ozone et/ou oxydants dissous est $C_i$ ;
b) on fabrique une solution mère d'eau ozonée, à teneur déterminée et constante en ozone $C_m$ ;
c) on introduit un prélèvement $V_m$ de la solution mère dans un échantillon de volume $V_i$ de ladite réserve;
d) on mesure, immédiatement après ajout et/ou à intervalle de temps déterminé (régulier ou non) après l'ajout, la teneur $C_f$ en ozone dissous et/ou en oxydants résiduels, dudit échantillon après ajout de $V_m$ ;
e) on détermine le taux de traitement à appliquer audit liquide en fonction de la valeur de la grandeur $C_f - C_i$ (c'est à dire en fonction de la différence entre la quantité d'ozone et d'oxydants résiduels présente dans l'échantillon après ajout et celle initialement présente dans l'échantillon avant ajout).

**Description**

La présente invention concerne le domaine du traitement à l'ozone de liquides, traitement à l'ozone qui peut être, on le sait, utilisé dans un très grand nombre d'applications industrielles : par exemple en liaison avec l'environnement (traitement des eaux et des effluents...), pour le traitement de produits alimentaires à l'ozone (liquides alimentaires notamment boissons, lavage de produits alimentaires à l'eau ozonée tels produits de la mer ou encore fruits et légumes), ou encore dans l'aquaculture et la pisciculture (élevages où l'on procède à un traitement à l'ozone de tout ou partie de l'eau qui alimente le ou les bassins d'élevage du site utilisateur).

En considérant ici le cas des élevages en pisciculture ou aquaculture, si le traitement à l'ozone de tels élevages a fait l'objet d'une littérature très abondante (on se reportera par exemple à la demande de brevet français au nom de la demanderesse FR-99 06567 qui s'intéresse à l'ozonation d'élevages fonctionnant en circuits fermés), littérature qui souligne l'intérêt considérable qu'il y a à traiter l'eau qui alimente les bassins (intérêt sanitaire, productivité...), reste qu'il subsiste des difficultés et des questions concernant la mise en oeuvre des traitements d'ozonation, liées notamment au fait que les espèces oxydantes résiduelles d'un traitement à l'ozone peuvent être toxiques pour les espèces élevées, qu'il s'agisse du résiduel d'ozone lui-même dans le milieu ou encore de tous les sous-produits oxydants provenant des réactions d'ozonation de l'ozone sur le milieu (en particulier des sels dans le cas de l'eau de mer).

Il faut également tenir compte du fait que lorsqu'on injecte de l'ozone dans de l'eau (ou tout autre liquide), une partie de cet ozone est immédiatement consommée par l'eau dans l'attaque de matières organiques telles que couleurs, bactéries, ou encore dans des réactions avec des sels présents dans l'eau, pour laisser place seulement ensuite, véritablement à un résiduel d'ozone ou d'oxydant qui peut être pris en compte pour effectuer le traitement visé par l'application considérée.

Le traitement visé pourra bien entendu être très varié selon cette application considérée et selon le cahier des charges recherché par chaque site utilisateur, de manière à réaliser l'une ou plusieurs des actions parmi les actions suivantes : une décoloration, une désinfection, une désodorisation, ou encore une purification de l'eau (élimination d'espèces toxiques ou indésirables).

L'homme du métier parle couramment, concernant cette consommation immédiate d'ozone par l'eau (ou par le liquide dans le cas de l'application la plus générale), de « demande en ozone » de l'eau considérée.

On évoque dans ce qui suit le cas du traitement d'une eau tout en gardant à l'esprit le fait que ces traitements (et la notion de « demande en ozone » qui est associée) s'appliquent de façon plus générale à des liquides d'origine très variée.

On peut donc dire concernant cette « demande en ozone » de l'eau à traiter :

- que cette demande représente la quantité d'ozone immédiatement consommée (on peut même dire "avalée") par l'eau ;
- en d'autres termes, cette « demande en ozone" est donc la quantité d'ozone que l'on peut introduire dans l'eau, avant l'apparition d'un résiduel (d'ozone et/ou d'oxydant) dans l'eau ;
- elle dépend de la qualité de l'eau à traiter ;
- la détermination de cette demande en ozone de l'eau à traiter, et de la rémanence du résiduel d'ozone ou d'oxydant consécutif, permet de calculer la dose de traitement en ozone à appliquer à l'eau considérée.

Le taux de traitement à appliquer à l'eau considérée sera donc déterminé en fonction :

- du résiduel d'ozone dissous ou d'oxydant à maintenir dans l'eau après avoir satisfait la demande instantanée de l'eau considérée, en d'autres termes de l'efficacité recherchée selon le traitement considéré (par exemple quantité à partir de laquelle on obtient effectivement l'effet désinfectant recherché) ;
- de la sécurité du lieu d'utilisation considéré (législation locale, ou encore par exemple dans le cadre des élevages de poisson ne tolérer aucun résiduel d'ozone et/ou d'oxydants au-delà d'un temps de séjour AT donné).

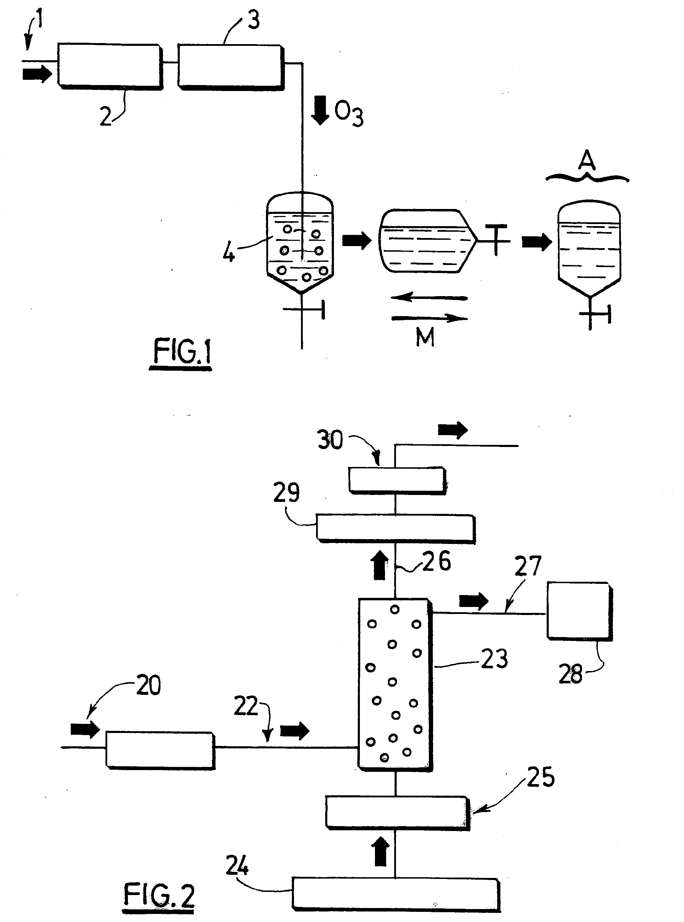

La littérature relate un certain nombre de solutions existantes pour la détermination de la demande en ozone d'une eau à traiter, qui seront davantage détaillées dans le cadre des figures 1 et 2 ci-après, la figure 1 étant consacrée à une solution de détermination que l'on peut qualifier de solution en mode « batch » alors que la figure 2 est consacrée à une solution de détermination que l'on peut qualifier de détermination « en continu ».

Selon la première méthode antérieure de détermination de la demande en ozone d'une eau à traiter, on injecte un gaz à teneur déterminée en ozone dans un échantillon de l'eau à traiter, on procède à une agitation de l'échantillon d'eau, puis d'une part à une détermination de la teneur en ozone dissous de l'eau, et d'autre part à une détermination de la teneur en ozone de la phase gazeuse se situant au-dessus de la phase liquide d'eau dans l'échantillon.

La demande en ozone est alors déterminée par une équation de conservation classique reliant la quantité initiale d'ozone injectée au travers du gaz, la teneur en ozone dissous de l'eau après agitation, et la teneur en ozone gazeux de la phase gaz existant au-dessus de la phase liquide de l'échantillon.

Les inconvénients d'une telle méthode de détermination en mode « batch » sont notamment liés à la précision nécessaire dans la connaissance de la teneur en ozone du gaz ozoné introduit, mais également au fait que pour doser l'échantillon (au niveau de sa phase liquide et de sa phase gazeuse) il faut prélever du liquide et du gaz, ce qui déjà en soi (rien que dans le fait de prélever) fausse le résultat de la mesure puisque de l'ozone s'échappe alors naturellement du milieu liquide.

Selon maintenant la seconde méthode de détermination dite « en continu », on fait arriver dans un contacteur de type colonne à bulles en continu l'eau à traiter d'une part, et un gaz ozoné d'autre part, pour réaliser au niveau du contacteur la dissolution, afin d'analyser en sortie de contacteur l'eau et son résiduel d'ozone dissous, ainsi que le gaz ozoné non transféré (gaz de sortie).

On conçoit alors que cette méthode de détermination en continu est certes fiable, et d'ailleurs très utilisée dans la littérature, mais présente incontestablement l'inconvénient de nécessiter un volume d'eau important, d'atteindre l'équilibre, mais également la mise en oeuvre de beaucoup de matériel (de nombreux analyseurs), d'être à ce titre relativement complexe, et très peu compacte.

La présente invention a notamment pour objectif d'apporter une solution aux problèmes techniques ci-dessus mentionnés en liaison avec les méthodes de détermination selon l'art antérieur.

La méthode de détermination selon l'invention d'un taux de traitement (T) à l'ozone d'un liquide à traiter comporte alors la mise en oeuvre des mesures suivantes :

a) on dispose d'une réserve dudit liquide à traiter dont la teneur initiale en ozone et/ou oxydants dissous est $C_i$,
b) on fabrique une solution mère d'eau ozonée (à partir d'eau distillée ou déminéralisée), à teneur donnée et constante en ozone dissous $C_m$ ;
c) on prélève un volume $V_m$ de la solution mère pour l'introduire dans un échantillon de volume $V_i$ de ladite réserve;
d) on mesure, immédiatement après ajout et/ou à intervalles de temps déterminés (réguliers ou non) après l'ajout, la teneur $C_f$ en ozone dissous et/ou en oxydants résiduels dudit échantillon après l'ajout de $V_m$ ;
e) on détermine le taux de traitement à appliquer audit liquide en fonction de la valeur de la grandeur $C_i$ - $C_f$ (donc on l'aura compris en fonction de la différence entre la quantité d'ozone et d'oxydants résiduels présente dans l'échantillon après ajout et celle initialement présente dans l'échantillon avant ajout).

Comme il apparaîtra clairement à l'homme du métier, la solution mère d'eau ozonée, pour être « à teneur donnée et constante en ozone dissous », sera avantageusement obtenue à saturation (ceci afin d'éviter au maximum les phénomènes d'auto décomposition) : la saturation d'un liquide en un gaz dissous -ici en ozone- étant on le sais, selon la loi de Henry, fonction de la nature du liquide, de la température, mais aussi de la pression partielle de l'ozone dans le gaz injecté.

Selon un des modes de mise en oeuvre de l'invention, ledit liquide à traiter est une eau douce, et l'on détermine le taux de traitement T de la façon suivante :

- on évalue la consommation en ozone de l'eau par l'expression :

$$\text{Consommation} = C_m\, V_m + C_i\, V_i - C_f\, (V_m + V_i) \text{ en mg ;}$$

- on en déduit la demande en ozone de ladite eau à traiter par l'expression suivante : demande ozone = consommation/$V_i$ en mg/l ;
- on choisit un taux de traitement T supérieur à la valeur de la demande ozone.

Comme il apparaîtra clairement à l'homme du métier à la lecture de tout ce qui précède, le taux de traitement T supérieur à la valeur de la demande ozone sera choisi en fonction de chaque application finale, et donc selon les cas en fonction du résiduel recherché par le site utilisateur, et en fonction de la rémanence d'ozone et/ou d'oxydant dans l'eau à traiter pour différents temps de séjour (voir étape d) ci-dessus).

Selon un des modes de mise en oeuvre de la méthode dans le cas d'une telle eau douce, on choisit le taux de traitement T supérieur à la valeur de la demande ozone de la façon suivante :

i) on connaît la valeur du résiduel d'ozone dissous et/ou d'oxydants résiduels recherché par l'application considéré pour ladite eau à traiter pour son utilisation finale;
ii) on évalue, pour l'application considérée de ladite eau à traiter, le temps de parcours minimum $\Delta T_{min}$ entre le point de traitement et le point utilisateur final;
iii) on évalue la rémanence de l'ozone dans l'eau à traiter en effectuant, conformément à l'étape d), des mesures, à intervalles de temps après l'ajout, de la teneur $C_f$ en ozone dissous et/ou en oxydants résiduels des échantillons après l'ajout de $V_m$ ;

iiii) on choisit un taux de traitement T en fonction : dudit résiduel d'ozone dissous et/ou d'oxydants résiduels recherché par l'application considérée, et de la rémanence $C_f$ mesurée durant l'étape précédente iii) après un intervalle $\Delta T_{min}$ après l'ajout de $V_m$.

Selon un autre des modes de mise en oeuvre de l'invention, ledit liquide à traiter est une eau de mer, et l'on procède alors par itérations successives pour déterminer le volume maximum de prélèvement $V_m^{max}$ permettant d'obtenir immédiatement après ajout, une valeur de la teneur $C_f$ sensiblement identique à celle de la teneur $C_i$, ceci de la façon suivante :

- si $C_f$ est supérieur à $C_i$, on réitère les étapes c) et d) précédemment listées avec un prélèvement de volume $V_n$, $V_n$ étant inférieur à $V_m$, jusqu'à obtenir une valeur de la teneur $C_f$ sensiblement identique à celle de la teneur $C_i$ (à environ 0,01 mg/l près) ;
- si $C_f$ est sensiblement identique à $C_i$, on réitère les étapes c) et d) avec un prélèvement de volume $V_n$, $V_n$ étant supérieur à $V_m$, tant que la valeur de la teneur $C_f$ est sensiblement identique à la teneur $C_i$ (à environ 0,01 mg/l près) ;
- la demande en ozone de l'eau à traiter correspond alors à $C_m V_m^{max} / V_i$ (en mg/l) ;
- on choisit un taux de traitement T égal à la demande en ozone.

Selon un autre des modes de réalisation de l'invention, ladite eau à traiter est une eau de mer, et on procède alors par itérations successives pour déterminer le volume maximum de prélèvement $V_m^{max}$, permettant d'obtenir, après un temps déterminé $\Delta T$ après ledit ajout, une valeur de la teneur $C_f$ sensiblement identique à celle de la teneur $C_i$, ceci de la façon suivante :

- si $C_f$ est supérieur à $C_i$, on réitère les étapes c) et d) précédemment listées avec un prélèvement de volume $V_n$, $V_n$ étant inférieur à $V_m$, jusqu'à obtenir, après ledit temps déterminé $\Delta T$, une valeur de la teneur $C_f$ sensiblement identique à celle de la teneur $C_i$ (à environ 0,01 mg/l près);
- si $C_f$ est sensiblement identique à $C_i$, on réitère les étapes c) et d) avec un prélèvement de volume $V_n$, $V_n$ étant supérieur à $V_m$, tant que la valeur de la teneur $C_f$ est, après ledit temps déterminé $\Delta T$, sensiblement identique à la teneur $C_i$ (à environ 0,01 mg/l près) ;
- la demande en ozone de l'eau à traiter correspond alors à $C_m V_m^{max} / V_i$ (en mg/l) ;
- on choisit un taux de traitement T égal à la demande en ozone, et l'on prévoit la présence d'un réacteur de rétention de l'eau ainsi traitée à l'aide dudit taux de traitement T, apte à retenir l'eau ainsi traitée pendant un temps d'au moins $\Delta T$, avant toute utilisation ultérieure de l'eau ainsi traitée.

La présente invention concerne également un procédé de traitement d'un liquide par injection dans le liquide à traiter d'un gaz ozoné, mettant en oeuvre un taux de traitement en ozone T, se caractérisant en ce que l'on détermine le taux de traitement T à appliquer audit liquide en appliquant la méthode de détermination précédemment décrite.

Selon un des aspects de l'invention, le procédé de traitement vise à réaliser l'une ou plusieurs des actions parmi les actions suivantes : une décoloration, une désinfection, ou encore une désodorisation de l'eau, et le liquide ainsi traité est ensuite utilisée pour le lavage de produits alimentaires ou dans des élevages d'aquaculture ou de pisciculture, élevages du genre où l'on procède à un traitement à l'ozone de tout ou partie de l'eau qui alimente le ou les bassins d'élevage du site utilisateur.

D'autres caractéristiques et avantages de l'invention ressortiront de la description suivante, donnée uniquement à titre illustratif et nullement limitatif, et faite en référence aux dessins annexés sur lesquels :

- la figure 1 est une illustration schématique d'une méthode de détermination de la demande en ozone selon l'art antérieur, qualifiée de méthode « batch » ;
- la figure 2 est une illustration schématique d'une méthode de détermination de la demande en ozone selon l'art antérieur, qualifiée de méthode « continue» ;
- la figure 3 est une illustration schématique d'un mode de réalisation de la méthode de détermination de la demande en ozone selon l'invention.

On reconnaît sur la figure 1 la présence d'une arrivée 1 de gaz oxygéné sur un ozoneur, apte à produire en sa sortie un gaz ozoné (analysé par l'analyseur 3), gaz qui est dirigé vers un échantillon d'eau 4 (de volume $V_i$) pour y être injecté.

L'échantillon subit ensuite au niveau de la phase symbolisée sur la figure par la lettre M une étape d'agitation (mélange), pour ensuite faire l'objet d'une analyse A :

- on détermine selon cette méthode la teneur en ozone dans le gaz ozoné issu de l'ozoneur (= $C_g(O3)$) d'où l'on déduit la quantité d'ozone transmise pendant un temps d'injection t (= $Q_g(O3)$);
- on détermine la teneur en ozone dissous dans l'eau après agitation (=$C_{eau}(O3)$) d'où la quantité d'ozone dissous dans l'échantillon $V_i$ considéré ($V_i$ x $C_{eau}(O3)$);
- on détermine la teneur en ozone dans la phase gazeuse présente au dessus de l'eau de l'échantillon (= $C_{ph.\ gaz}(O3)$) d'où la quantité d'ozone présent dans cette phase gazeuse (= $Q_{ph.\ gaz}(O3)$);
- on détermine enfin la CONSOMMATION en ozone de cet échantillon par l'équation de conservation : $Q_g(O3) = V_i$ $C_{eau}(O3)$ + $Q_{ph.\ gaz}(O3)$ + CONSOMMATION en mg;
- la « demande en ozone » de l'eau testée, en mg/l s'exprime alors par l'expression : demande ozone = CONSOMMATION/$V_i$.

On peut alors faire les remarques suivantes sur cette méthode :

- elle nécessite une grande précision sur la quantité de gaz ozoné introduit ;
- l'équation de conservation repose sur un prélèvement de la phase gaz et un prélèvement de la phase liquide de l'échantillon, ce qui crée en soi un effet qualifié par l'homme du métier de «stripping» (de l'ozone dissous s'échappe du liquide pour rejoindre la phase gazeuse), faussant ainsi d'autant les résultats obtenus.

On reconnaît sur la figure 2 la présence d'une arrivée d'eau 20 (dont il faut déterminer la demande en ozone), qui transite par un analyseur d'ozone dissous 21, avant d'atteindre un contacteur 23 (par exemple du type colonne à bulles), où il rencontre un gaz ozoné en provenance de l'ensemble ozoneur 24/analyseur de gaz 25.

L'eau 27 en sortie de contacteur est envoyée sur un analyseur d'ozone dissous 28, tandis que le gaz non dissous 26 qui sort du contacteur est dirigé vers un ensemble composé d'un analyseur 29 et un système de detoxification 30 avant d'être évacué vers l'extérieur.

Le processus de détermination de la « demande en ozone » selon cette méthode met alors en oeuvre les étapes suivantes :

- on détermine selon cette méthode (grâce à l'analyseur 21) la quantité d'ozone dissous dans l'eau initiale et donc la quantité d'ozone dissous entrant par unité de temps ($Q_{eau}(O3)_i$ en mg/mn);
- on détermine grâce à l'analyseur 25 la quantité d'ozone dans le gaz en provenance de l'ozoneur 24, et donc la quantité d'ozone gazeux entrant par unité de temps ($Q_{gaz}(O3)_i$ en mg/mn);
- on détermine grâce à l'analyseur 28 la quantité d'ozone dissous dans l'eau de sortie du contacteur et donc la quantité d'ozone dissous par unité de temps ($Q_{eau}(O3)_f$ en mg/mn);
- on détermine grâce à l'analyseur 29 la quantité d'ozone dans le gaz de sortie du contacteur, et donc la quantité d'ozone gazeux par unité de temps ($Q_{gaz}(O3)_f$ en mg/mn);
- on détermine alors la consommation en ozone de cette eau en continu par l'équation de conservation :

$$\text{CONSOMMATION} = Q_{eau}(O3)_i + Q_{gaz}(O3)_i - Q_{eau}(O3)_f - Q_{gaz}(O3)_f \text{ en mg/mn.}$$

- la « demande en ozone » de l'eau testée, en mg/l s'exprime alors par l'expression : demande = CONSOMMATION (mg/mn)/débit d'eau (l/mn).

On peut alors faire les remarques suivantes sur cette méthode :

- on note déjà les difficultés techniques liées au caractère continu du montage (volume d'eau, temps d'atteinte de l'équilibre du système, distribution des temps de séjour dans la colonne à bulle...);
- l'équipement analytique nécessaire (4 équipements !) est très lourd et onéreux ;
- la méthode dans son ensemble est de ce fait complexe et peu compacte.

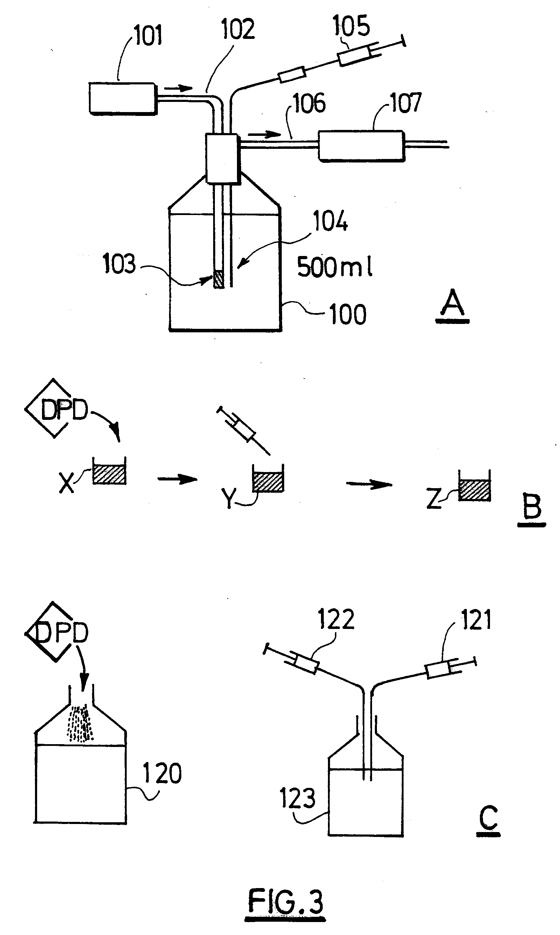

On va illustrer dans ce qui suit la méthode de détermination de la « demande en ozone » d'une eau à traiter selon la présente invention, grâce à la figure 3. Dans tout ce qui suit, les dosages d'ozone et/ou d'oxydants dissous dans l'eau se font par utilisation d'un spectrophotomètre HACH DR 2010 reposant sur un dosage du chlore total par le réactif coloré DPD (mesure effectuée à 530 nm).

On a illustré au niveau de la figure 3A la fabrication de la solution mère, obtenue ici à partir de 500 ml d'eau déminéralisée.

Dans un flacon 100 (contenant les 500 ml initiaux d'eau déminéralisée), on note la présence :

- d'une ligne 102 d'arrivée de gaz ozoné en provenance d'un ozoneur 101, la ligne 102 étant munie en son extrémité d'un diffuseur poreux 103 ;
- d'une ligne capillaire 104, permettant le prélèvement d'un volume $V_m$ de solution mère au travers d'une seringue 105 (mode de prélèvement choisi afin d'éviter la mise en contact avec l'air ambiant et conserver ainsi une concentration $C_m$ constante à la solution mère) ;
- d'une ligne 106 d'évacuation de la phase gaz non dissoute, en passant par un système de detoxification.

Comme déjà signalé plus haut dans le cadre de la présente description, on procède avantageusement à une saturation de la solution mère aux conditions de pression et température pratiquées, le gaz ozoné arrive alors continuellement pendant toute la durée des opérations.

On considère au niveau de la figure 3B différentes opérations subies par un échantillon d'eau à traiter :

- étape X : échantillon témoin d'eau à traiter (ici 25 ml) dont on dose la teneur initiale en ozone et/ou oxydants résiduels dissous (réactif coloré DPD);
- étape Y : échantillon d'eau à traiter (ici 23 ml) auquel on ajoute par une seringue un prélèvement $V_m$ (ici 2 ml) de solution mère (figure A) ;
- étape Z : l'échantillon en provenance de l'étape Y (après ajout) est dosé en ozone et/ou oxydants résiduels dissous (réactif coloré DPD). Le dosage intervient immédiatement après ajout de la solution mère ou bien après un intervalle de temps déterminé. On pourra ainsi, pour suivre la teneur du résiduel en ozone et/ou oxydants résiduels avec le temps, fabriquer au stade Y plusieurs échantillons, afin de les doser ultérieurement en Z à intervalles de temps i. e temps de séjour (ex : immédiatement après ajout, à 1, 2, 3, 5, 10 mn etc....).

La figure 3C fournit quant à elle un détail permettant de mieux visualiser les étapes de la figure 3B :

- pour l'étape X : le flacon 120 d'échantillon témoin d'eau à traiter dont on dose la teneur initiale en ozone et/ou oxydants résiduels dissous avec ajout du réactif coloré DPD ;
- pour l'étape Y : le flacon 123 comportant un échantillon d'eau à traiter (23 ml) auquel on ajoute par la seringue 121 un prélèvement $V_m$ = 2 ml de solution mère, et dont on dose ensuite, au temps voulu (étape Z), le résiduel d'ozone et/ou d'oxydants résiduels dissous grâce à l'ajout du réactif coloré DPD (seringue 122).

Comme on l'aura compris à la lumière de ce qui précède, la méthode de détermination de la « demande en ozone » d'un liquide à traiter selon la présente invention apporte des avantages significatifs par rapport aux méthodes antérieures ci-dessus rappelées :

- la méthode ne nécessite pas de connaître les caractéristiques initiales du gaz ozoné utilisé, seules important les caractéristiques connues et stables de la solution mère ;
- l'ajout de solution mère, l'ajout de réactif DPD, l'analyse spectrophotométrique sont effectués dans une cuve échantillon unique (celle du matériel spectrophotométrique utilisé), sans nécessiter de transvasement, de prélèvement de phases liquides et gazeuses, et donc en évitant les erreurs et artefacts liés aux manipulations signalées plus haut dans le cas de l'art antérieur ;
- c'est précisément le mérite de la présente invention d'avoir pensé et montré qu'il était avantageux de passer par l'ajout d'une phase liquide calibrée et contrôlée pour déterminer la « demande en ozone » du liquide à traiter, et partant de là le taux de traitement à appliquer.

Un premier exemple de mise en oeuvre de la méthode de détermination de la demande en ozone selon l'invention a été effectué sur de l'eau douce de rivière (pour une application de pisciculture fonctionnant en circuit ouvert) dans les conditions expérimentales suivantes :

a) fabrication de la solution mère à partir d'eau distillée : on fabrique une solution saturée à $C_m$ = 8,9 mg O3/l;

b) teneur initiale ($C_i$) en oxydants d'un échantillon de 25 ml de l'eau douce à traiter : 0,007 mg/l (mesure spectrophotomètre : 0,01 mg $Cl_2$/l) ;

c) on ajoute, à un échantillon $V_i$ de 23 ml d'eau à traiter, $V_m$ = 2 ml de solution mère d'eau ozonée (2ml de solution mère comportent donc 0,018 mg d'ozone) ;

d) on mesure après ajout du réactif DPD la teneur en oxydants dans le volume total de 25 ml : $C_f$ = 0,71 mg $O_3$/l, donnant donc 0,018 mg d'ozone présents dans cet échantillon de 25 ml (mesure spectrophotomètre : 1,05 mg

Cl2/l) ;

e) on en déduit que l'ozone n'a pas été consommé, on retrouve en effet la totalité de l'ozone qui avait été ajouté au travers des 2 ml de solution mère, on en conclue que la « demande instantanée en ozone » de cette eau douce est négligeable. Ce résultat est en bon accord avec le fait que l'eau à traiter était de bonne qualité, naturellement peu chargée.

f) pour l'installation de pisciculture considérée qui utilise cette eau douce, le temps de séjour minimum $\Delta T_{min}$ (temps de parcours) entre le point de traitement et le point d'utilisation était de 20 mn, et l'on recherchait pour les poissons un résiduel d'ozone nul après ce temps $\Delta T_{min}$ ;

g) on a alors estimé la rémanence de l'ozone dans l'eau à traiter en effectuant des mesures sur 3 échantillons par ajout de réactif DPD à t=5 mn, t=10mn, et t=20 mn après l'ajout de $V_m$ (en conservant bien entendu chaque échantillon bouché et à l'abri de la lumière durant chaque temps de séjour testé).

On obtient alors les résultats suivants :

| t(mn) | O3 résiduel(mg/l) | consommation(%) |
|-------|-------------------|-----------------|
| 0 | 0,71 | /// |
| 5 | 0,21 | 70 |
| 10 | 0,05 | 93 |
| 20 | 0,01 | 100 |

h) on détermine alors le taux de traitement T en fonction d'une part de l'objectif de désinfection visé, et d'autre part des conditions de sécurité des poissons en assurant un résiduel d'ozone nul après 20 minutes : un taux de traitement $\leq$ 0,7 mg 03/1 convient donc bien pour un tel cahier des charges.

Un <u>second exemple</u> de mise en oeuvre de la méthode de détermination de la demande en ozone selon l'invention a été effectué sur de l'eau de mer d'un élevage de poissons fonctionnant en circuit fermé, l'exemple ayant été réalisé dans les conditions expérimentales suivantes :

a) fabrication de la solution mère à partir d'eau distillée : on fabrique une solution saturée à $C_m$ = 5,4 mg O3/l (mesure spectrophotomètre : 0,64 mg Cl2/l) ;

b) teneur initiale en oxydants d'un échantillon témoin de 25 ml de l'eau de mer à traiter : $C_i$ = 0,045 mg/l;

c) les résultats obtenus (mesure du résiduel d'oxydants totaux 1mn après l'ajout d'un prélèvement $V_m$ de solution mère), sont rassemblés dans le tableau ci-après où les symboles utilisés ont la signification suivante :

$V_m$ : ml de solution mère ajoutés à un échantillon $V_i$ d'eau de mer à traiter (tels que $V_i + V_m$ = 25 ml);

T : taux de traitement = mg d'ozone ajoutés (via le prélèvement) par litre d'eau de mer à traiter (Vi) ;

C : résultat de la mesure de résiduel d'oxydants totaux (en mg/l) dans le flacon de 25 ml.

| $V_m$ (ml) | T(mg/l) | C(mg/l) |
|-----------|---------|---------|
| 5,6 | 1,20 | 0,12 |
| 4,2 | 0,90 | 0,11 |
| 3,2 | 0,70 | 0,08 |
| 2,5 | 0,55 | 0,08 |
| 1,5 | 0,33 | 0,06 |
| 1,2 | 0,26 | 0,05 |

On déduit de ce tableau le fait qu'un taux de traitement pour cette eau de mer circulant en circuit fermé de 0,33 mg O3/l conviendrait à cette application de pisciculture en assurant une absence de résiduel oxydants ($C_f$ sensiblement identique à $C_i$, à environ 0,01 milligramme/l près).

Si l'on décidait d'adopter un taux de traitement supérieur à 0,33 mg O3/l d'eau (par exemple 0,55 ou encore 0,70), il faudrait se baser sur une mesure de la rémanence des oxydants, et prévoir la présence d'une capacité de rétention de l'eau traitée pendant quelques minutes permettant la disparition du résiduel avant tout contact ultérieur de l'eau ainsi traitée avec les poissons.

Il est à noter que l'on pourrait également, tout en adoptant un taux de traitement supérieur à 0,33 mg O3/l d'eau, ne pas choisir la solution d'une capacité de rétention mais adopter en revanche une méthode d'abattement du résiduel d'ozone et d'oxydants de l'eau après traitement, ceci par l'ajout d'un réactif tel un composé de la famille des bisulfites, ou encore par passage sur un filtre à charbon actif.

**Revendications**

1. Méthode de détermination d'un taux de traitement à l'ozone d'un liquide à traiter, selon laquelle on procède aux mesures suivantes :

   a) on dispose d'une réserve dudit liquide à traiter dont la teneur initiale en ozone et/ou oxydant dissous est $C_i$ ;

   b) on fabrique une solution mère d'eau ozonée à teneur donnée et constante en ozone dissous $C_m$ ;

   c) on prélève un volume $V_m$ de la solution mère pour l'introduire dans un échantillon de volume $V_i$ de ladite réserve;

   d) on mesure, immédiatement après ajout et/ou à intervalles de temps déterminés, réguliers ou non, après l'ajout, la teneur $C_f$ en ozone dissous et/ou en oxydants résiduels dissous dudit échantillon après l'ajout de $V_m$ ;

   e) on détermine le taux de traitement à appliquer audit liquide en fonction de la valeur de la grandeur $C_f - C_i$.

2. Méthode selon la revendication 1 caractérisée en ce que ledit liquide à traiter est une eau douce, et en ce que l'on détermine alors le taux de traitement de la façon suivante :

   - on évalue la consommation de l'ozone par ladite eau à traiter par l'expression : CONSOMMATION = $C_m V_m + C_i V_i - C_f (V_m + V_i)$ en mg ;
   - on en déduit la demande en ozone de ladite eau à traiter par l'expression suivante : demande ozone=CONSOMMATION / $V_i$ en mg/l
   - on choisit un taux de traitement T supérieur à la valeur de la demande ozone.

3. Méthode selon la revendication 2 caractérisée en ce que l'on choisit ledit taux de traitement T supérieur à la valeur de la demande ozone de la façon suivante :

   i) on connaît la valeur du résiduel d'ozone dissous et/ou d'oxydants résiduels recherché par l'application considéré pour ladite eau à traiter dans son utilisation finale;

   ii) on évalue, pour l'application considérée de ladite eau à traiter, le temps de parcours minimum $\Delta T_{min}$ entre le point de traitement et le point utilisateur final;

   iii) on évalue la rémanence de l'ozone dans l'eau à traiter en effectuant, conformément à l'étape d), des mesures, à intervalles de temps après l'ajout, de la teneur $C_f$ en ozone dissous et/ou en oxydants résiduels des échantillons après l'ajout de $V_m$ ;

   iiii) on choisit un taux de traitement T en fonction : dudit résiduel d'ozone dissous et/ou d'oxydants résiduels recherché par l'application considérée, et de la rémanence $C_f$ mesurée durant l'étape précédente iii) après un intervalle $\Delta T_{min}$ après l'ajout de $V_m$.

4. Méthode selon la revendication 1 caractérisée en ce que ledit liquide à traiter est une eau de mer, et en ce que l'on procède par itérations successives pour déterminer le volume maximum $V_m{}^{max}$ dudit prélèvement permettant d'obtenir immédiatement après ajout une valeur de la teneur $C_f$ sensiblement identique à celle de la teneur $C_i$, de la façon suivante :

   - si $C_f$ est supérieure à $C_i$, on réitère les étapes c) et d) avec un prélèvement de volume $V_n$, $V_n$ étant inférieur à $V_m$, jusqu'à obtenir une valeur de la teneur $C_f$ sensiblement identique à celle de la teneur $C_i$ ;
   - si $C_f$ est sensiblement identique à $C_i$, on réitère les étapes c) et d) avec un prélèvement de volume $V_n$, $V_n$ étant supérieur à $V_m$, tant que la valeur de la teneur $C_f$ est sensiblement identique à la teneur $C_i$ ;
   - on choisit un taux de traitement T égal à $C_m V_m{}^{max}/V_i$.

5. Méthode selon la revendication 1 caractérisée en ce que ledit liquide à traiter est une eau de mer, et en ce que on procède alors par itérations successives pour déterminer le volume maximum de prélèvement $V_m{}^{max}$, permettant d'obtenir, après un temps déterminé $\Delta T$ après ledit ajout, une valeur de la teneur $C_f$ sensiblement identique à celle de la teneur $C_i$, de la façon suivante :

   - si $C_f$ est supérieur à $C_i$, on réitère les étapes c) et d) précédemment listées avec un prélèvement de volume $V_n$, $V_n$ étant inférieur à $V_m$, jusqu'à obtenir, après ledit temps déterminé $\Delta T$, une valeur de la teneur $C_f$ sensiblement identique à celle de la teneur $C_i$ ;

- si $C_f$ est sensiblement identique à $C_i$, on réitère les étapes c) et d) avec un prélèvement de volume $V_n$, $V_n$ étant supérieur à $V_m$, tant que la valeur de la teneur $C_f$ est, après ledit temps déterminé $\Delta T$, sensiblement identique à la teneur $C_i$ ;
- on choisit un taux de traitement T égal à $C_m V_m^{max} / V_i$ et l'on prévoit la présence d'une capacité de rétention de l'eau ainsi traitée à l'aide dudit taux de traitement T, apte à retenir l'eau ainsi traitée pendant un temps d'au moins $\Delta T$, avant toute utilisation ultérieure de l'eau ainsi traitée.

6. Procédé de traitement d'un liquide par injection dans le liquide à traiter d'un gaz ozoné, à l'aide d'un taux de traitement en ozone T, caractérisé en ce que l'on détermine le taux de traitement T à appliquer audit liquide en appliquant la méthode de détermination selon l'une des revendications 1 à 5.

7. Procédé de traitement selon la revendication 6, caractérisé en ce que le traitement vise à réaliser l'une ou plusieurs des actions parmi les actions suivantes : une décoloration, une désinfection, ou encore une désodorisation, et en ce que le liquide ainsi traité est ensuite utilisé pour le lavage de produits alimentaires ou dans des élevages d'aquaculture ou de pisciculture, élevages du genre où l'on procède à un traitement à l'ozone de tout ou partie de l'eau qui alimente le ou les bassins d'élevage du site utilisateur.

FIG.1

FIG.2

101    102         105

104
500 ml

103    100

A

DPD

X    Y    Z    B

DPD

120

122    121

123    C

FIG.3

**Office européen des brevets**

# RAPPORT DE RECHERCHE EUROPEENNE

Numéro de la demande

EP 00 40 2957

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 5 324 666 A (TEUTSCHER MICHAEL ET AL) 28 juin 1994 (1994-06-28) * abrégé; figure 1 * | 1,6 | G01N33/18 |
| A | US 4 409 183 A (FISCHER BERTRAM) 11 octobre 1983 (1983-10-11) * abrégé * | 1 | |
| A | US 5 403 602 A (ENDICO FELIX W) 4 avril 1995 (1995-04-04) * abrégé * | 1 | |

| DOMAINES TECHNIQUES RECHERCHES (Int.Cl.7) |
|---|
| G01N |

Le présent rapport a été établi pour toutes les revendications

| Lieu de la recherche | Date d'achèvement de la recherche | Examinateur |
|---|---|---|
| LA HAYE | 23 mars 2001 | Duchatellier, M |

CATEGORIE DES DOCUMENTS CITES

X : particulièrement pertinent à lui seul
Y : particulièrement pertinent en combinaison avec un autre document de la même catégorie
A : arrière–plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**                   EP 00 40 2957

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

23-03-2001

| Document brevet cité au rapport de recherche | | Date de publication | Membre(s) de la famille de brevet(s) | | Date de publication |
|---|---|---|---|---|---|
| US 5324666 | A | 28-06-1994 | DE | 3827578 A | 15-02-1990 |
| | | | AT | 93058 T | 15-08-1993 |
| | | | DE | 58905291 D | 16-09-1993 |
| | | | WO | 9001696 A | 22-02-1990 |
| | | | EP | 0428514 A | 29-05-1991 |
| | | | JP | 4500555 T | 30-01-1992 |
| US 4409183 | A | 11-10-1983 | DE | 2928324 A | 29-01-1981 |
| US 5403602 | A | 04-04-1995 | US | 5328706 A | 12-07-1994 |

EPO FORM P0460

Pour tout renseignement concernant cette annexe : voir Journal Officiel de l'Office européen des brevets, No. 12/82